(19) **Europäisches Patentamt / European Patent Office / Office européen des brevets**

(11) **EP 1 463 443 B1**

(12) **EUROPÄISCHE PATENTSCHRIFT**

(45) Veröffentlichungstag und Bekanntmachung des Hinweises auf die Patenterteilung:
**23.07.2008 Patentblatt 2008/30**

(21) Anmeldenummer: **02795119.3**

(22) Anmeldetag: **07.12.2002**

(51) Int Cl.:
***A61B 5/00*** *(2006.01)*

(86) Internationale Anmeldenummer:
**PCT/EP2002/013884**

(87) Internationale Veröffentlichungsnummer:
**WO 2003/055382 (10.07.2003 Gazette 2003/28)**

(54) **VERFAHREN UND VORRICHTUNG ZUR BESTIMMUNG EINES LICHTTRANSPORTPARAMETERS UND EINES ANALYTEN IN EINER BIOLOGISCHEN MATRIX**

METHOD AND DEVICE FOR DETERMINING A LIGHT TRANSPORT PARAMETER AND AN ANALYTE IN A BIOLOGICAL MATRIX

PROCEDE ET DISPOSITIF DE DETERMINATION D'UN PARAMETRE DE TRANSPORT DE LUMIERE ET D'UN ANALYTE DANS UNE MATRICE BIOLOGIQUE

(84) Benannte Vertragsstaaten:
**AT BE BG CH CY CZ DE DK EE ES FI FR GB GR IE IT LI LU MC NL PT SE SI SK TR**

(30) Priorität: **22.12.2001 DE 10163972**

(43) Veröffentlichungstag der Anmeldung:
**06.10.2004 Patentblatt 2004/41**

(73) Patentinhaber:
• **Roche Diagnostics GmbH**
  **68305 Mannheim (DE)**
  Benannte Vertragsstaaten:
  **DE**
• **Hoffman-La Roche AG**
  **4070 Basel (CH)**
  Benannte Vertragsstaaten:
  **AT BE BG CH CY CZ DK EE ES FI FR GB GR IE IT LI LU MC NL PT**

(72) Erfinder:
• **KRAEMER, Uwe**
  **68549 Ilvesheim (DE)**
• **HEIN, Heinz-Michael**
  **64295 Darmstadt (DE)**
• **HERMANN, Marcus**
  **24159 Kiel (DE)**

(74) Vertreter: **Pfeifer, Hans-Peter et al**
**Durm & Partner**
**Patentanwälte**
**Beiertheimer Allee 19**
**76137 Karlsruhe (DE)**

(56) Entgegenhaltungen:
| | |
|---|---|
| **EP-A- 0 843 986** | **WO-A-01/09589** |
| **WO-A-94/10901** | **DE-A- 10 110 599** |
| **DE-A- 19 630 381** | **US-A- 5 452 723** |
| **US-A- 5 630 423** | **US-A- 5 867 807** |
| **US-B1- 6 219 566** | |

• **MARQUEZ G ET AL: "White light oblique incidence reflectometer for measuring absorption and reduced scattering spectra of tissue-like turbid media" OPTICS EXPRESS, 22 DEC. 1997, OPT. SOC. AMERICA, USA, Bd. 1, Nr. 13, XP002239731 ISSN: 1094-4087 in der Anmeldung erwähnt**
• **SHAO-POW LIN ET AL: "Measurement of tissue optical properties by the use of oblique-incidence optical fiber reflectometry" APPLIED OPTICS, 1 JAN. 1997, OPT. SOC. AMERICA, USA, Bd. 36, Nr. 1, Seiten 136-143, XP002239732 ISSN: 0740-3224**

**Beschreibung**

Die Erfindung betrifft ein Verfahren zur selektiven Bestimmung eines für die Lichtstreuung in einer biologischen Matrix charakteristischen Lichttransportparameters, insbesondere zur nichtinvasiven Bestimmung der Konzentration eines die Lichtstreuung in der biologischen Matrix beeinflussenden Analyten, insbesondere von Glucose in der biologischen Matrix. Auch eine hierfür geeignete Vorrichtung ist Gegenstand der Erfindung.

Der Begriff "biologische Matrix" bezeichnet eine Körperflüssigkeit oder ein Gewebe eines lebenden Organismus. Biologische Matrices, auf die sich die Erfindung bezieht, sind optisch heterogen, d.h. sie enthalten eine Vielzahl von Streuzentren, an denen eingestrahltes Licht gestreut wird. Im Falle von biologischem Gewebe, insbesondere Hautgewebe, werden die Streuzentren von den Zellwänden und anderen in dem Gewebe enthaltenen festen Bestandteilen gebildet. Körperflüssigkeiten, insbesondere Blut, sind ebenfalls optisch heterogene biologische Matrices, weil sie Partikel enthalten, an denen Licht vielfach gestreut wird.

Der Transport von Licht in einer biologischen Matrix wird im wesentlichen durch die Lichtstreuung an in der Matrix enthaltenen Streuzentren und durch die optische Absorption bestimmt. Physikalische Größen, die diese beiden Eigenschaften quantitativ beschreiben, werden als Lichttransportparameter (Streuparameter beziehungsweise Absorptionsparameter) bezeichnet. Unter einem Streuparameter in diesem Sinne ist in erster Linie der Streukoeffizient $\mu_s$ und unter einem Absorptionsparameter in erster Linie der optische Absorptionskoeffizient $\mu_a$ zu verstehen. Es ist allerdings im Rahmen der Erfindung nicht erforderlich, daß diese Parameter quantitativ in den gebräuchlichen Maßeinheiten bestimmt werden. Vielmehr ist es das Ziel der Erfindung, reproduzierbar und selektiv einen Parameter zu ermitteln, der die optische Streuung in der biologischen Probe unabhängig von deren optischer Absorption beschreibt. Nachfolgend wird ohne Beschränkung der Allgemeinheit auf den Streukoeffizienten $\mu_s$ als Beispiel für einen Streuparameter Bezug genommen.

Die selektive Bestimmung des Streukoeffizienten ist in medizinischer Hinsicht generell von Interesse, weil aus der Wechselwirkung des Lichts mit Hautgewebe und anderen biologischen Matrizes diagnostisch interessante Informationen abgeleitet werden können. Beispielsweise ist es in der Dermatologie möglich, auf diesem Wege Hauteigenschaften zu charakterisieren.

Von besonderer Bedeutung ist die Untersuchung des Streuverhaltens einer biologischen Matrix zum Zwecke der nichtinvasiven Bestimmung der Konzentration von die Lichtstreuung beeinflussenden Analyten, insbesondere Glucose. Der Zusammenhang zwischen der Glucosekonzentration und der Lichtstreuung in biologischen Matrices wird in der EP 0659055 B1 beschrieben. Wie darin (und in zahlreichen anderen Publikationen, die sich mit der Analyse von Glucose im menschlichen Körper befassen) erläutert wird, ist die Qualität der Therapie von Diabetikern entscheidend davon abhängig, daß der zeitliche Verlauf des Blutzuckerspiegels in ihrem Körper sehr häufig, nach Möglichkeit kontinuierlich, bestimmt wird. Dadurch können schwerwiegende Spätschäden des Diabetes Mellitus, wie Erblindung oder schwere Durchblutungsstörungen, die zur Amputation von Gliedmaßen führen können, vermieden werden. Die wünschenswerte kontinuierliche Beobachtung des Blutzuckerspiegels ist mit den konventionellen invasiven Methoden (bei denen ein Blutstropfen aus dem Körper des Patienten gewonnen und mit einem heutzutage in guter Qualität zu günstigen Kosten erhältlichen Analysesystem ausgewertet wird) nicht möglich. Es hat deshalb schon zahlreiche Versuche gegeben, die Konzentration der Glucose auf nichtinvasivem Wege zu bestimmen. Eine nähere Darlegung hierzu ist der genannten europäischen Patentschrift zu entnehmen.

Bei dem in der EP 0659055 B1 beschriebenen Verfahren wird zur Bestimmung eines Glucosewertes eine Mehrzahl von "Detektionsmessungen" durchgeführt, bei denen jeweils Licht als Primärlicht an einem definierten Einstrahlungsort in die biologische Matrix eingestrahlt wird, das Licht in der biologischen Matrix entlang einem Lichtweg propagiert und ein Intensitätsmeßwert von an einem definierten Detektionsort austretendem Sekundärlicht gemessen wird. Aus der Abhängigkeit des Intensitätsmeßwertes von dem Meßabstand zwischen dem jeweiligen Einstrahlungsort und dem jeweiligen Detektionsort wird in einem Auswerteschritt mittels eines Auswertealgorithmus und einer Kalibration die Glucosekonzentration ermittelt.

Die überraschende Erkenntnis, daß mit einem derartigen Meßverfahren der Verlauf der Glucosekonzentration in Hautgewebe oder einer anderen biologischen Matrix gemessen werden kann, wird in der EP 0659055 B1 damit erklärt, daß die mit der Änderung der Glucosekonzentration verbundene Änderung des Brechungsindex der in der Matrix enthaltenen Flüssigkeit (obwohl sehr klein) zu einer Änderung der Lichtstreuung führt, die zur Bestimmung der Glucosekonzentration verwendet werden kann, wenn man das Streuverhalten des Lichts unter Beachtung der in der europäischen Patentschrift gegebenen Hinweise untersucht. Gemäß einer bevorzugten Ausführungsform sollen in dem Auswerteschritt die Einflüsse der Absorption und der Streuung durch Auswertung der Intensitätsverteilung des Sekundärlichts als Funktion des Abstandes des Detektionsortes von dem Einstrahlungsort getrennt werden.

Auch in der wissenschaftlichen Literatur wird bereits seit längerem diskutiert, $\mu_a$ und $\mu_s$ aus der Abhängigkeit I($\rho$) der Intensität I des Sekundärlichts von dem Meßabstand $\rho$ (nachfolgend als "Intensitätsprofil" bezeichnet) zu bestimmen. Als theoretische Grundlagen dienen dabei die Diffusionstheorie sowie numerisch-statistische Verfahren (Monte Carlo-Rechnungen). Die Theorie bildet ein Modell zur Beschreibung des Lichtausbreitungsverhaltens in einer streuenden Matrix, durch das ein mathematischer Zusammenhang zwischen dem Intensitätsprofil I(p) und den in dem Modell verwendeten Modellparametern (vor allem den Lichttransportparametern $\mu_a$ und $\mu_s$ und der Intensität des eingestrahlten Primärlichts $I_0$) hergestellt wird. Im Prinzip ist es möglich, die Lichttransportparameter dadurch zu bestimmen, daß man einen Fit durchführt, bei dem durch Variation der Modellparameter das theoretisch berechnete Intensitätsprofil optimal an experimentelle Ergebnisse angepaßt wird. Hierzu kann beispielsweise auf folgende Publikationen verwiesen werden:

1) T.J. Farrell et al.: "A diffusion theory model of spatially resolved, steady-state diffuse reflectance for the noninvasive determination of tissue optical properties in vivo", Med. Phys. 19, 879 bis 888 (1992)

2) R.C. Haskell et al.: "Boundary conditions for the diffusion equation in radiative transfer", J. Opt.Soc.Am A, 11,2727 bis 2741 (1994).
Obwohl darin über eine gute Übereinstimmung von Meßwerten und theoretischen Berechnungen berichtet wird, haben diese Verfahren keine praktische Bedeutung für die Bestimmung der Gluocosekonzentration in einer biologischen Matrix erlangt.
In der Patentliteratur sind verschiedene Verfahren beschrieben, deren Ziel es ist, in einer biologischen Matrix $\mu_a$ und $\mu_s$ zu bestimmen, um daraus analytische Daten für medizinische Zwecke, insbesondere zur Bestimmung der Glucosekonzentration, zu gewinnen:

3) Gemäß der EP 0760091 B1 werden für jeweils mindestens zwei unterschiedliche Meßlichtwege jeweils mindestens zwei Frequenzdomänen-spektroskopische Messungen durchgeführt, bei denen jeweils die Phasenverschiebung des Sekundärlichts gegenüber dem Primärlicht sowie ein Intensitätsmeßwert (nämlich die DC-Intensität oder die AC-Intensität) bestimmt wird. Aus diesen mindestens vier Meßwerten wird ein Absorptionsparameter und/oder ein Streuparameter abgeleitet. Frequenzdomänen-Meßverfahren arbeiten mit im GHz-Bereich moduliertem Licht und bedingen dadurch einen großen meßtechnischen Aufwand.

4) In der EP 0774658 A2 ist ein Verfahren beschrieben, bei dem zur Analyse der Streueigenschaften einer biologischen Matrix die Reflexionseigenschaften an der Oberfläche der Matrix variiert werden. Beispielsweise kann die Kontaktfläche des für die Messung verwendeten Meßkopfes unterschiedliche Teilbereiche mit unterschiedlicher Reflektivität aufweisen. Auf diese Weise werden bei zwei Meßabständen die Reflexionseigenschaften mindestens zweifach variiert. In der Publikation wird dargelegt, daß diese mindestens vier Meßwerte verwendet werden können, um (entweder auf Basis der Diffusionstheorie oder empirisch-numerisch) Absorption und Streuung zu trennen. Auch dieses Verfahren ist jedoch relativ aufwendig. Außerdem ist es schwierig, die für die Analyse der Glucosekonzentration erforderliche Reproduzierbarkeit der Meßwerte zu erreichen.

5) In der deutschen Patentschrift 10110599 wird ein spezieller Auswertealgorithmus zur Ermittlung des Streukoeffizienten aus einer Mehrzahl von Detektionsmessungen beschrieben. Dabei wird aus der relativen zeitlichen Änderung des Intensitätsprofils ein Zeitableitungswert als Zwischenwert berechnet, um die Trennung der Einflüsse der Modellparameter (insbesondere des Absorptionskoeffizienten und des Streukoeffizienten) zu erleichtern und dadurch auf einfache Weise eine verbesserte Genauigkeit bei der Ermittlung des Streukoeffizienten zu erreichen.

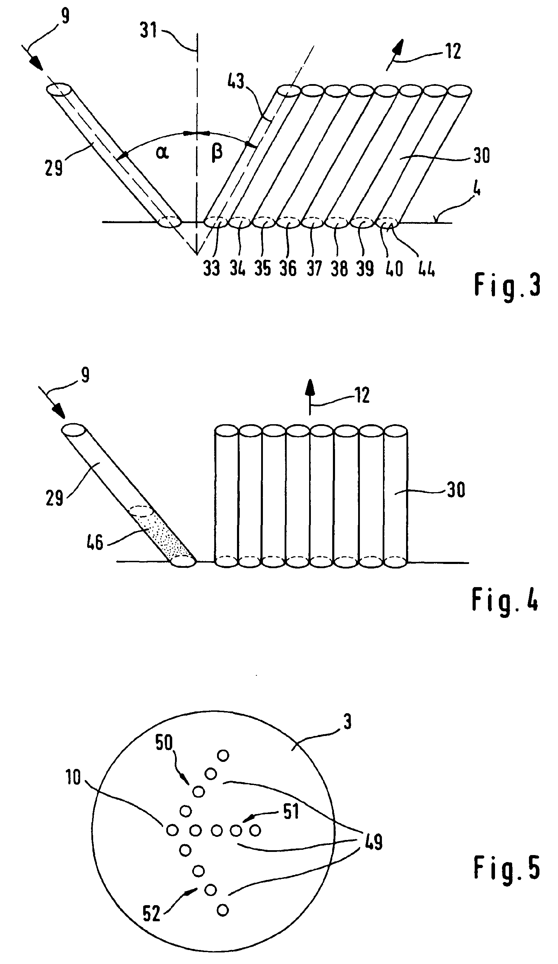

Auf Basis dieses Standes der Technik liegt der Erfindung die Aufgabe zugrunde, in einer biologischen Matrix den Streukoeffizienten $\mu_s$ (oder einen anderen die Lichtstreuung beschreibenden Parameter) beziehungsweise die Konzentration eines die Lichtstreuung in der biologischen Matrix beeinflussenden Analyten selektiv mit einem Verfahren zu bestimmen, das sich durch einfache Handhabung, einen geringen apparativen Aufwand und hohe Genauigkeit auszeichnet.
Diese Aufgabe wird gemäß gelöst durch ein Verfahren gemäß Anspruch 1.
Die Erprobung einer erfindungsgemäßen Meßanordnung mit der Kombination dieser Merkmale hat gezeigt, daß sie auf einfache Weise eine genaue selektive Ermittlung des Streukoeffizienten ermöglicht. Vor allem ergibt sich bei in vivo-Untersuchungen an der Hautoberfläche zur Bestimmung der Glucosekonzentration eine verbesserte Korrelation des Meßwertes mit konventionell (invasiv) bestimmten Analysewerten.
Durch die schräge Einstrahlung wird eine geringe Eindringtiefe des Primärlichts in die biologische Matrix bei gleichzeitig ausreichend langem Lichtweg erreicht. Im Rahmen der Erfindung wurde festgestellt, daß die damit verbundene verbesserte Homogenität des untersuchten Gewebes zu einem genaueren Meßresultat beiträgt und außerdem eine verbesserte Korrelation der in der Haut nachgewiesenen Glucosekonzentration mit der im Blut gemessenen Glucosekonzentration festzustellen ist.
Die schräge Einstrahlung von Licht ist ein für sich genommen bekanntes Konstruktionsmerkmal. Beispielsweise wird in dem US-Patent 5,630,423 ein Primärlichtstrahl unter einem Winkel zwischen 5 und 85° eingestrahlt, um aus der durch die schräge Einstrahlung verursachten Verschiebung des Schwerpunktes der Intensitätsverteilung des Sekundärlichts eine Information über die Streueigenschaften der Probe zu gewinnen. Aus der Verteilungskurve der Intensitätsverteilung (in Figur 3 des US-Patentes mit M bezeichnet) wird eine Mittelwertkurve C abgeleitet, die gegenüber der 0-Position horizontal um einen Wert $\Delta x$ verschoben ist. In dem US-Patent wird ein mathematischer Zusammenhang zwischen diesem $\Delta x$ und $\mu_s$ angegeben, mittels dessen der reduzierte Streukoeffizient $\mu_s'$ berechnet wird. Die schräge Einstrahlung dient somit einem ganz anderen Zweck als bei der vorliegenden Erfindung.

Die Erfindung unterscheidet sich von dem Verfahren des US-Patentes auch insofern, als dort das Primärlicht kontaktlos mit einer Freistrahlanordnung eingestrahlt und das Sekundärlicht (ebenfalls kontaktlos) mittels einer CCD-Kamera detektiert wird. Grundlage der Auswertung sind dabei Messungen mit Meßabständen bis zu mehr als 1,3 cm. Dies entspricht in Hautgewebe nahezu dem zwanzigfachen der mittleren freien Weglänge (ca. 0,7 mm). Bei dem erfindungsgemäßen Verfahren ist es hingegen notwendig, daß das Primärlicht mittels eines die Oberfläche der biologischen Matrix, beispielsweise also die Hautoberfläche, kontaktierenden Lichtleitelementes eingestrahlt wird. Auch die Detektion des Sekundärlichtes erfolgt vorzugsweise mittels eines die Matrix kontaktierenden Lichtleitelementes.

Der Begriff "Lichtleitelement" umfaßt dabei unterschiedliche Konstruktionen, beispielsweise in Form von lichtleitenden Stäben oder Fasern. Auch ein transparenter Abschnitt ("Fenster") in einer Platte, die einen Meßkopf auf einer die Haut kontaktierenden Seite abschließt (Hautkontaktplatte), ist ein Lichtleitelement in diesem Sinne. Erforderlich ist jedenfalls, daß das Licht zumindest auf dem letzten Lichtwegabschnitt vor dem Eindringen in die Matrix in einem transparenten Festkörper transportiert wird, der in Kontakt mit der Oberfläche der biologischen Matrix steht.

Die Funktion der vorliegenden Erfindung erfordert, daß mindestens zwei, bevorzugt mindestens drei und besonders bevorzugt mindestens vier der in dem Auswerteschritt zur Ermittlung des Streukoeffizienten verwendeten Detektionsmessungen mit Meßabständen durchgeführt werden, die höchstens der fünffachen mittleren freien Weglänge entsprechen. Besonders bevorzugt werden in dem Auswerteschritt ausschließlich Intensitätsmeßwerte verwendet, die aus Detektionsmessungen resultieren, bei denen der Meßabstand höchstens der zehnfachen mittleren freien Weglänge entspricht. Die genannten Grenzwerte des Meßabstandes betragen in Haut etwa 3,5 mm beziehungsweise 7 mm.

Eine Weiterentwicklung des in dem US-Patent 5,630,423 beschriebenen Verfahrens ist beschrieben in

G. Marquez et al. "White light oblique incidence reflectometer for measuring absorption and reduced scattering spectra of tissue-like turbid media", OPTICS EXPRESS, 1997, 454-460.

Darin wird das Licht unter einem Winkel von 45° mittels Lichtleitfasern eingestrahlt und detektiert. Die Einstrahlung erfolgt simultan mit einer Wellenlänge-Bandbreite von 256 nm. Dadurch soll es unter Berücksichtigung weiterer Annahmen möglich sein, nicht nur $\mu_s$, sondern auch $\mu_a$ aus $\Delta x$ und der Strahlrichtung (Einstrahlungswinkel $\alpha$) zu ermitteln.

Die Ermittlung des Streukoeffizienten in dem Auswerteschritt basiert bei den genannten Publikationen auf der Diffusionstheorie. Bei der Erfindung wird hingegen vorzugsweise eine numerische Simulation verwendet, wobei die theoretische Abhängigkeit der Intensität I des Sekundärlichts von dem Meßabstand $\rho$ und den Lichttransportparameters $\mu_a$ und $\mu_s$ mittels einer numerischen Simulation, beispielsweise eine Monte-Carlo-Rechnung, errechnet und ein Fit durchgeführt wird, bei dem die Lichttransportparameter so variiert werden, daß die numerische Simulation die experimentell bestimmten Werte der Intensität I in Abhängigkeit von dem Meßabstand $\rho$ optimal beschreibt. Die Verwendung einer numerischen Simulation ermöglicht es, Meßsignale auszuwerten, die mit sehr kurzen Meßabständen von einer bis einigen wenigen mittleren freien Weglängen gewonnen werden, für die die Diffusionstheorie keine korrekten Voraussagen liefert.

Aus den so gefundenen Meßwerten des Streukoeffizienten kann durch Kalibration eine entsprechende Information über die Konzentration eines die Lichtstreuung in der biologischen Matrix beeinflussenden Analyten abgeleitet werden. Alternativ besteht auch die Möglichkeit, ohne explizite Berechnung eines die Lichtstreuung charakterisierenden Lichttransportparameters eine unmittelbare numerische Korrelation zwischen den gemessenen Intensitäten und der Analytkonzentration (mittels Kalibration) zu ermitteln.

Vorzugsweise wird in dem Auswerteschritt ein Auswertealgorithmus mit folgenden Teilschritten durchgeführt:

a) Ermittlung der theoretischen Abhängigkeit der Intensität (I) des Sekundärlichts von dem Meßabstand ($\rho$) und der optischen Absorption ($\mu_a$) mittels einer numerischen Simulation für einen typischen Wert ($\mu_{s,MC}$) der optischen Streuung,

b) Speicherung des Ergebnisses der Simulation in Form einer Lookup-Tabelle, durch die vorgegebenen Werten des Meßabstands (p) und der optischen Absorption ($\mu_a$) jeweils ein Intensitätswert ($I(\rho,\mu_a)$) zugeordnet wird,

c) Bestimmung des für die Lichtstreuung in der optischen Matrix charakteristischen Parameters ($\mu_s$) mittels eines Fit-Verfahrens, bei dem für eine Vielzahl unterschiedlicher Werte der den Lichttransport in der biologischen Matrix charakterisierenden Lichttransportparameter ($\mu_a,\mu_s$) die Abhängigkeit der Intensität (I) von dem Meßabstand (p) berechnet und mit den bei den Detektionsmessungen gemessenen Intensitätsmeßwerten verglichen wird, um die Werte der Lichttransportparameter ($\mu_a,\mu_s$) zu bestimmen, bei denen eine optimale Übereinstimmung der rechnerisch bestimmten Abhängigkeit der Intensität (I) von dem Meßabstand (p) mit den entsprechenden Intensitätsmeßwerten erreicht wird, wobei der Schritt c) folgende Teilschritte umfaßt:

c1) Berechnen eines Skalierungsfaktors (s) aus der Relation einer beliebigen optischen Streuung ($\mu_s$), zu der in dem Schritt a) verwendeten typischen optischen Streuung ($\mu_{s,MC}$),

c2) Umformen der unabhängigen Variablen ($\rho, \mu_a$) der in dem Schritt b) gespeicherten Lookup-Tabelle mittels des Skalierungsfaktors (s),

c3) Berechnen der Abhängigkeit der Intensität (I) von der optischen Absorption ($\mu_a$) und dem Meßabstand (p) bei dem gewählten Wert der optischen Streuung ($\mu_s$) mittels der umgeformten Variablen auf Basis der in der Lookup-Tabelle abgespeicherten Werte.

Durch Verwendung dieses Auswertealgorithmus wird es möglich, die Auswertung auf Basis numerischer Simulationsverfahren auch bei verhältnismäßig kleinen Analysegeräten einzusetzen, die nur mit relativ geringer Computerleistung ausgestattet sind. Dies gilt insbesondere für tragbare Analysegeräte, wie sie von Diabetikern zur regelmäßigen Bestimmung ihrer Glucosewerte verwendet werden. Dabei werden vorzugsweise die Schritte a) und b) des Auswertealgorithmus außerhalb des tragbaren Analysegerätes mittels eines leistungsfähigen Computersystems durchgeführt, während der Schritt c) mit den Teilschritten $c_1$ bis $c_3$ in dem tragbaren Analysegerät mit einer wesentlich geringeren Computerleistung durchgeführt werden kann.

Die Erfindung wird nachfolgend anhand von in den Figuren dargestellten Ausführungsbeispielen näher erläutert. Die darin beschriebenen Besonderheiten können einzeln oder in Kombination verwendet werden, um bevorzugte Ausgestaltungen der Erfindung zu schaffen. Es zeigen:

Fig. 1    eine schematische Querschnittsdarstellung einer Vorrichtung für die optische Analyse einer biologischen Matrix,

Fig. 2    eine schematische perspektivische Darstellung der für die Einstrahlung des Primärlichts und die Detektion des Sekundärlichts verwendeten Lichtleitelemente gemäß einer ersten Ausführungsform der Erfindung,

Fig. 3    eine schematische perspektivische Darstellung der für die Einstrahlung des Primärlichts und die Detektion des Sekundärlichts verwendeten Lichtleitelemente gemäß einer zweiten Ausführungsform der Erfindung,

Fig. 4    eine schematische perspektivische Darstellung der für die Einstrahlung des Primärlichts und die Detektion des Sekundärlichts verwendeten Lichtleitelemente gemäß einer dritten Ausführungsform der Erfindung.

Fig. 5    eine Aufsicht auf die Unterseite eines Meßkopfes gemäß einer weiteren bevorzugten Ausführungsform.

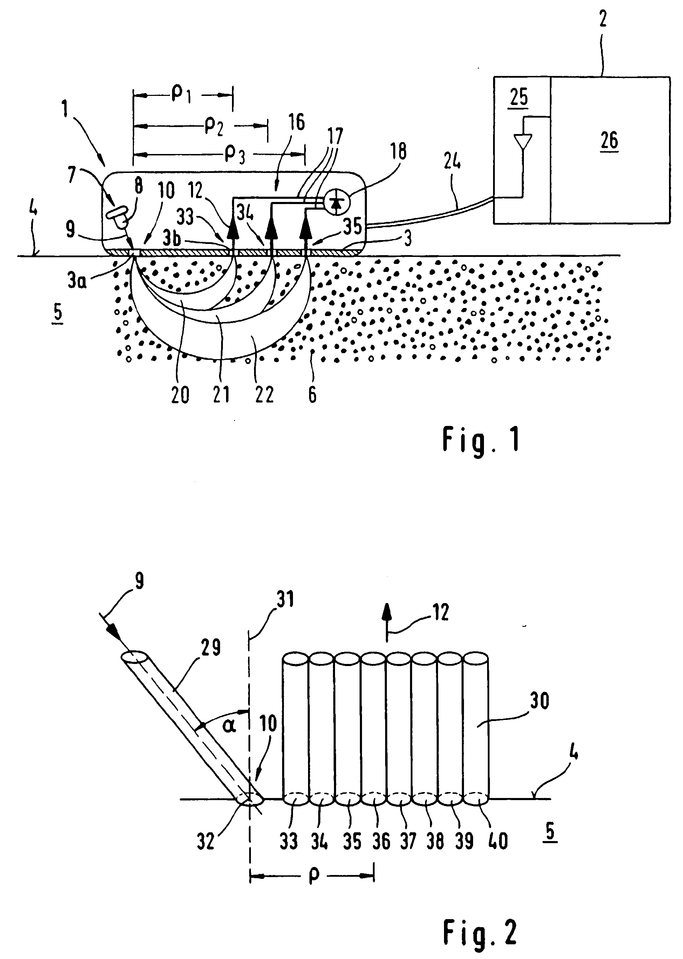

Die in Figur 1 stark schematisiert dargestellte Vorrichtung zur selektiven Bestimmung von $\mu_s$ in einer biologischen Matrix besteht im wesentlichen aus einem Meßkopf 1 und einer Signalverarbeitungs- und Auswerteeinheit 2. Nachfolgend wird auf Hautgewebe als Beispiel für die biologische Matrix Bezug genommen.

Der Meßkopf 1 liegt mit der Unterseite einer Hautkontaktplatte 3 auf einer Oberfläche 4 der Haut 5 auf, die eine Vielzahl von Streuzentren 6 enthält. Im Inneren des Meßkopfes 1 befinden sich Lichteinstrahlungsmittel 7 mit einer Leuchtdiode 8, die dazu dienen, Primärlicht (Pfeil 9) durch ein Einstrahlungsfenster 3a an einem Einstrahlungsort 10 in die biologische Matrix 5 einzustrahlen.

Durch Pfeile 12 symbolisiertes Sekundärlicht, das an drei von Detektionsfenstern 3b der Hautkontaktplatte 3 definierten Detektionsorten 33, 34 und 35 austritt, wird von insgesamt mit 16 bezeichneten Detektionsmitteln detektiert. Die Detektionsmittel 16 schließen bei der dargestellten Ausführungsform Lichtleitfasern 17 ein, durch die das Sekundärlicht aller drei Detektionsorte einem gemeinsamen Fotoempfänger 18 (beispielsweise einer Fotodiode, insbesondere Avalanche-Fotodiode 18, zugeführt wird. Um die notwendige Trennung der Intensitätsmeßwerte der drei Detektionsorte zu ermöglichen, enthalten die Lichtleitfasern 17 nicht dargestellte optische Schalter.

Die Lichtwege, längs der das in die biologische Matrix 5 eingestrahlte Licht zwischen dem Einstrahlungsort 11 und dem Detektionsort 12 bis 14 propagiert, sind in Figur 1 symbolisch dargestellt und mit 20 bis 22 bezeichnet. Infolge der Streuung in der biologischen Matrix lassen sich selbstverständlich keine scharf begrenzten Lichtwege angeben. Es ist jedoch davon auszugehen, daß die meisten der als Sekundärlicht detektierten Photonen näherungsweise auf einem gekrümmten Lichtweg - ähnlich wie dargestellt - propagieren, wobei die mittlere Eindringtiefe mit der Größe des Meßabstandes $\rho$ zwischen Einstrahlungsort 10 und Detektionsort 33 bis 35 zunimmt.

Das Ausgangssignal des Fotoempfängers wird über ein Kabel 24 einer Signalverarbeitungselektronik 25 zugeführt. Dort wird es in üblicher Weise verstärkt, aufbereitet und digitalisiert, so daß an ihrem Ausgang in digitaler Form Intensitätsmeßwerte zur Verfügung stehen, die der Intensität des an den Detektionsorten 33 bis 35 austretenden Sekundärlichts entsprechen.

Insoweit ist die dargestellte Vorrichtung konventionell und muß deshalb nicht näher erläutert werden. Sowohl die Einstrahlungsmittel als auch die Detektionsmittel können in Form von in die Hautkontaktplatte 3 integrierten Lichtsendern beziehungsweise lichtempfindlichen Elementen oder mit Hilfe von Lichtleitfasern realisiert sein, die das Licht von einem weiter entfernten Lichtsender zu der Hautkontaktplatte 3 hinführen beziehungsweise von dieser zu einem Lichtempfänger transportieren. Erforderlich ist bei jeder Ausführungsform der Erfindung, daß in Kontakt zu der Oberfläche 4 ein Lichtleitelement vorhanden ist. Es kann beispielsweise auch durch das transparente Gehäuse einer in die Hautkontaktplatte 3 integrierten Leuchtdiode gebildet werden.

Die unterschiedlichen Meßabstände können durch unterschiedliche Kombinationen von Einstrahlungs- und Detektionsorten realisiert sein. Insbesondere können die in Figur 1 dargestellten drei Meßabstände $\rho_1$, $\rho_2$ und $\rho_3$ im Prinzip auch dadurch realisiert sein, daß an drei unterschiedlichen Einstrahlungsorten eingestrahlt und an einem Detektionsort gemessen wird.

Weitere Einzelheiten zur Konstruktion des Meßkopfes, zur Durchführung der Detektionsmessungen und zur Messung der Intensitätsmeßwerte für unterschiedliche Meßabstände können dem publizierten Stand der Technik entnommen werden. Dabei kann insbesondere auf die EP 0659055 B1 verwiesen werden, in der unterschiedliche Anordnungen und Konstruktionen der Lichteinstrahlungsmittel und der Detektionsmittel beschrieben sind.

Der Meßkopf 1 und die Signalverarbeitungselektronik 25 sind jedenfalls so ausgebildet, daß von der Signalverarbeitungselektronik 25 Intensitätsmeßwerte für die bei dem jeweiligen Meßkopf möglichen Meßabstände (im dargestellten Fall die Meßabstände $\rho_1$, $\rho_2$ und $\rho_3$) bestimmt und in digitaler Form an die Auswerteelektronik 26 weitergeleitet werden. Die Auswerteelektronik 26 enthält - wie bei digitalen Geräten üblich - einen Mikrocomputer, der die weiter unten beschriebenen Berechnungen durchführt.

Figur 2 verdeutlicht die geometrische Anordnung der Lichtleitelemente gemäß einer ersten bevorzugten Ausführungsform der Erfindung. Das Primärlicht 9 wird mittels eines Einstrahlungslichtleitelementes 29, dessen optische Achse unter einem Winkel $\alpha$ zu einem Lot 31 auf die Oberfläche 4, der im dargestellten Fall etwa 40° beträgt (allgemein jedoch zwischen 5 und 85° liegen kann), in die Haut 5 eingestrahlt. Das Lichtleitelement 29 kann aus einer und mehreren Lichtleitfasern bestehen. Seine auf der Oberfläche 4 der Haut 5 aufliegende Stirnfläche 32 ist derartig schräg geschliffen, daß sie bei dem gegebenen Neigungswinkel $\alpha$ flach auf der Oberfläche 4 aufliegt.

Das Sekundärlicht 12 wird bei der in Figur 2 dargestellten Ausführungsform mit Hilfe von acht Detektionslichtleitelementen 30 detektiert, die die Hautoberfläche 4 an acht Detektionsorten 33 bis 40 kontaktieren. Die Detektionsorte 33 bis 40 befinden sich in Meßabständen $\rho$ (dargestellt ist der Meßabstand des Detektionsortes 36) von dem Einstrahlungsort 10. Die Lichtleitelemente 29 sind senkrecht zu der Oberfläche 4 (also parallel zu dem Lot 31) orientiert, so daß sie Sekundärlicht erfassen, das in Richtung des Lots 31 aus der Oberfläche 4 austritt.

Die in Figur 3 dargestellte Ausführungsform unterscheidet sich von Figur 2 hinsichtlich der Detektionsrichtung $\beta$, unter der die Detektionslichtleitelemente 30 das an den Detektionsorten 33 bis 40 aus der Oberfläche 4 austretende Sekundärlicht erfaßt. Sie verläuft schräg zu der Oberfläche 4, wobei der Winkel $\beta$ zwischen der Detektionsrichtung (optische Achse 43 der Detektionslichtleitelemente 29) und dem Lot 31 verschieden von Null ist und bevorzugt zwischen 5° und 85° liegt. Der Winkel $\beta$ ist vorzugsweise so orientiert, daß die Detektionsrichtung 43 auf den Einstrahlungsort 10 zugerichtet ist, d.h. die Winkel $\alpha$ und $\beta$ befinden sich auf verschiedenen Seiten des Lots 31. Die Stirnflächen 44 der Detektionslichtleiter 30 sind ebenso wie die Stirnfläche 32 des Einstrahlungslichtleitelementes 29 schräg poliert, so daß sie flach auf der Oberfläche 4 der Haut 5 aufliegen.

Mit einer solchen Anordnung wird das effektive Meßvolumen des Detektors, also das Volumen, durch das das von dem Detektor erfaßte Licht mit hoher Wahrscheinlichkeit propagiert ist, gegenüber einer senkrechten Detektion nach oben, zur Hautoberfläche hin, verschoben. Dies kann sinnvoll sein, um sensitiver in bestimmten Schichten der Haut zu messen.

Um bei flachen Einstrahlungswinkeln (Winkel $\alpha$ größer als etwa 30°) ein genauer definiertes Strahlprofil am Einstrahlungsort 10 zu erhalten, kann es zweckmäßig sein, das Primärlicht 9 mittels einer GRID-Linse 46 zu fokussieren, die bei der in Figur 4 dargestellten Ausführungsform in das Einstrahlungslichtleitelement 29 integriert ist.

Die Zuführung des Primärlichts 9 und die Weiterleitung des Sekundärlichts 12 kann ebenso wie die Lichtleitelemente 29 und 30 auf ganz unterschiedliche Wiese realisiert sein. Wichtig im Rahmen der vorliegenden Erfindung ist nur, daß mindestens der Einstrahlungswinkel $\alpha$, bevorzugt auch der Detektionswinkel $\beta$ in einem Winkelbereich zwischen 5° und 85° zu einem Lot 31 auf die Oberfläche 4 verläuft, die Lichtleitelemente 29,30 die Oberfläche 4 kontaktieren und die Meßabstände $\rho$ im Vergleich zu den bisher bekannten Verfahren sehr klein sind, wobei sie vorzugsweise innerhalb der weiter oben genannten Grenzen liegen.

Figur 5 zeigt die Unterseite einer Hautkontaktplatte 3 eines Meßkopfes, bei welchem gemäß einer weiteren bevorzugten Ausführungsform das Sekundärlicht an mehreren, jeweils eine Mehrzahl von Detektionsorten 49 umfassenden Detektionsfeldern 50, 51 und 52 detektiert wird. Dabei ist die Detektionsrichtung bei der Mehrzahl der Detektionsorte eines Detektionsfeldes einheitlich, jedoch bei der Mehrzahl der Detektionsfelder 50, 51, 52 unterschiedlich. Im dargestellten Fall sind die Detektionsmittel der Detektionsfelder 50, 51 und 52 jeweils aus verschiedenen Richtungen auf einen Einstrahlungsort 10 gerichtet. Eine Mehrzahl von in verschiedenen Richtungen zu einem gemeinsamen Einstrahlungsort 10 hin ausgerichteten Detektionsfelder kann dazu beitragen, die Photonenstatistik zu erhöhen und durch Redundanz ein stabileres Meßergebnis zu bewirken. Dadurch werden Fehler durch Inhomogenitäten der Hautoberfläche (beispielsweise Haarwurzeln oder Schweißdrüsen) ausgeschlossen.

Gemäß einer vorteilhaften Ausgestaltung können die Lichtleitelemente 29,30 in eine auf der Haut aufliegende Faserplatte integriert sein. Die Verwendung einer Faserplatte zur Messung eines Lichttransportparameters ist in dem US-Patent 5,893,364 beschrieben. Um eine schräge Einstrahlung des Lichts in die Haut zu erreichen, kann die Faserplatte schräg durchbohrt und ein Einstrahlungslichtleitelement in die schräge Bohrung eingesetzt werden. Alternativ kann auch ein Umlenkprisma in den Strahlengang eingesetzt werden, um das Licht in der gewünschten Richtung abzulenken. Die Detektion an einer Mehrzahl von Detektionsorten kann durch die Fasern der Faserplatte erfolgen, wie in dem genannten US-Patent beschrieben. Eine schräge Detektionsrichtung läßt sich realisieren, indem die optische Faserplatte unter einem gewünschten Winkel zur Richtung ihrer Lichtleitfasern schräg geschnitten wird.

Nachfolgend wird der gemäß dem zweiten Hauptaspekt der Erfindung verwendete Auswertealgorithmus näher erläutert.

Bei Abwesenheit von optischer Absorption läßt sich für die Streuung folgende Skalierungsrelation angeben:

$$(1) \quad I(\rho, \mu_s, \mu_a = 0) = \left(\frac{\mu_s}{\mu_{s,MC}}\right)^3 \cdot I\left(\rho \cdot \frac{\mu_s}{\mu_{s,MC}}, \mu_{s,MC}, \mu_a = 0\right)$$

Dabei wird von einer isotropen Matrix ausgegangen, so daß das Intensitätsprofil nur von dem Abstand vom Einstrahlungsort, nicht von dem Azemutwinkel φ abhängt. $\mu_{s,MC}$ ist ein fester Wert des Streukoeffizienten, von dem die Skalierung ausgeht.

Die Gleichung (1) besagt, daß das Intensitätsprofil I(ρ) bei Abwesenheit von Absorption mit der mittleren freien Weglänge $1/\mu_s$ skaliert. Auch die Weglänge, die die Photonen in einer absorptionsfreien streuenden Matrix zurücklegen, skaliert mit dem gleichen Faktor.

Für die optische Absorption gilt, daß die Absorptionswahrscheinlichkeit der Photonen gemäß dem Gesetz von Lambert-Beer nur von dem Absorptionskoeffizienten und der Länge des von dem Photon in der Matrix zurückgelegten Weges abhängt. Das absorptionsbedingte Intensitätsprofil ist deshalb eine expotentiell fallende Funktion, wobei allerdings berücksichtigt werden muß, daß die Koeffizienten dieser Funktion von $\mu_s$ und ρ abhängen. Zur Beschreibung des Intensitätsprofils in Abhängigkeit von der Absorption läßt sich folgende Gleichung verwenden:

$$(2) \quad I(\mu_a) = I_0 \exp(-a\mu_a - b\sqrt{\mu_a})$$

In den Schritten a und b des Auswertealgorithmus wird auf Basis einer numerischen Simulation eine Lookup-Tabelle berechnet. Speziell ist eine Monte-Carlo-Simulation geeignet. Es handelt sich dabei um eine Standard-Simulationsmethode, die insbesondere in der biomedizinischen Optik verwendet wird. Sie wird beispielsweise in der Publikation S.A. Prahl et al. "A Monte Carlo Model of light propagation in tissue", SPIE Institute Series, IS 5; 102-111, 1989 beschrieben. Bei einer derartigen Monte-Carlo-Simulation werden die Wege einer großen Anzahl von Photonen (typischerweise etwa $10^7$) simuliert, indem für jedes Photon auf Basis der seine Ausbreitung in einer streuenden und absorbierenden Matrix beschreibenden Gesetze ein zufälliger Pfad durch die Matrix bestimmt wird. Dabei wird insbesondere ermittelt, wo das Photon die Matrix verläßt und wie lange sein Weg durch die Matrix war. Hieraus resultiert für einen bestimmten Satz der Lichttransportparameter und eine bestimmte Geometrie der Matrix ein Intensitätsprofil I(ρ) des an der Oberfläche austretenden Lichts.

Als Grundlage der Monte-Carlo-Simulation wird von einem typischen Wert des Streukoeffizienten $\mu_{s,MC}$ und als bekannt vorausgesetzten Werten für den mittleren Brechungsindex n, den Anisotropiefaktor g und den Einstrahlungswinkel α ausgegangen. Zur Berechnung der numerischen Verteilung $I(\rho, \mu_s, \mu_a)$ wird die Oberfläche der Matrix in Teilbereiche aufgeteilt. Als Ergebnis der Simulationsrechnung erhält man eine zweidimensionale Tabelle von I-Werten in Abhängigkeit von $\rho_i$ und $\mu_{a,k}$.

Für die verschiedenen Werte der Absorption sind keine separaten Simulationen notwendig. Vielmehr kann die Absorptionswahrscheinlich für jedes Photon auf Basis von dessen Pfadlänge für verschiedene Werte von $\mu_a$ ausgerechnet und durch ein entsprechendes "Gewicht" des Photons ausgedrückt werden. Zur Datenreduktion und um schnellere Berechnungen zu ermöglichen, wird für jeden Teilbereich der Oberfläche ein nichtlinearer Least-Squares-Fit ausgeführt, wobei aus den errechneten Werten bei verschiedenen Absorptionen die Parameter a und b der Gleichung (2) ermittelt werden.

Auf diese Weise erhält man für jeden in der Rechnung verwendeten Meßabstand $\rho_i$ Werte der Intensität $I(\rho_i)$ und der Koeffizienten der Gleichung (2) $a(\rho_i)$ und $b(\rho_i)$, die in Form einer Lookup-Tabelle abgespeichert werden. Diese Lookup-Tabelle wird in dem Schritt c des Algorithmus (mit Detailschritten c1 bis c3) beispielsweise folgendermaßen verwendet:

Für jeden Wert von $\mu_s$, für den ein Intensitätsprofil benötigt wird, wird ein Skalierungsfaktor s berechnet gemäß $s = \mu_{s,MC}/\mu_s$.

Die Werte von $\rho_i$ werden mit diesem Faktor skaliert:

$$\rho_i' = \rho_i \cdot s.$$

Die Skalierung von $\rho$ führt zu einer veränderten Fläche der in der Simulation verwendeten Teilbereiche der Matrixoberfläche. Entsprechend müssen die Intensitätswerte angepaßt werden: $I_0'(\rho_i') = I_0(\rho_i)/s^2$.

Die Abschwächung durch die Absorption wird dadurch berücksichtigt, daß $\mu_a$ skaliert wird gemäß $\mu_a' = \mu_a \cdot s$. Die resultierenden Werte $\mu_a$ und $\rho_i$ werden in die Gleichung (2) eingesetzt, um ein theoretisches Intensitätsprofil $I(\rho_i, \mu_s, \mu_a)$ zu berechnen. Sofern die dabei verwendeten Werte von $\rho_i$ nicht mit den Meßabständen übereinstimmen, wird eine Interpolation durchgeführt.

Diese Berechnungen werden für eine Vielzahl von Werten der Parameter $\mu_s$, $\mu_a$ und $\rho$ durchgeführt und mit den gemessenen Werten verglichen. Die optimalen Werte der Parameter werden mittels eines nichtlinearen Least-Square-Fit ermittelt.

**Patentansprüche**

1. Verfahren zur selektiven Bestimmung eines für die Lichtstreuung in einer biologischen Matrix charakteristischen Lichttransportparameters, umfassend

   mindestens drei Detektionsmessungen, bei denen jeweils Licht als Primärlicht (9) an einem Einstrahlungsort (10) in die biologische Matrix (5) eingestrahlt wird, das Licht in der biologischen Matrix (5) entlang einem Lichtweg (20-22) propagiert und ein Intensitätsmeßwert von an einem Detektionsort (33-40), der sich bei den Detektionsmessungen in unterschiedlichen Meßabständen ($\varrho$) von dem jeweiligen Einstrahlungsort befindet, austretendem Sekundärlicht (12) gemessen wird, und

   einen Auswerteschritt, bei dem der für die Lichtstreuung in der biologischen Matrix (5) charakteristische Lichttransportparameter mittels eines Auswertealgorithmus aus den bei den Detektionsmessungen gemessenen Intensitätsmeßwerten abgeleitet wird,

   bei welchem

   das Primärlicht mittels eines die Oberfläche (4) der biologischen Matrix (5) an dem Einstrahlungsort (10) kontaktierenden Lichtleitelementes (29) eingestrahlt wird,

   das Primärlicht an dem Einstrahlungsort (10) unter einem Einfallswinkel ($\alpha$) zu einem Lot (31) auf die Oberfläche (4) zwischen 5° und 85° schräg in die biologische Matrix (5) eingestrahlt wird und

   bei mindestens zwei Detektionsmessungen der Meßabstand ($\varrho$) zwischen dem jeweiligen Einstrahlungsort (10) und dem jeweiligen Detektionsort (33-40) höchstens der fünffachen mittleren freien Weglänge des in der biologischen Matrix propagierenden Lichtes entspricht.

2. Verfahren zur Bestimmung eines Analyten, der die Lichtstreuung in einer biologischen Matrix beeinflußt, insbesondere von Glucose, umfassend

   mindestens drei Detektionsmessungen, bei denen jeweils Licht als Primärlicht (9) an einem Einstrahlungsort (10) in die biologische Matrix (5) eingestrahlt wird, das Licht in der biologischen Matrix (5) entlang einem Lichtweg (20-22) propagiert und ein Intensitätsmeßwert von an einem Detektionsort (33-40), der sich bei den Detektionsmessungen in unterschiedlichen Meßabständen ($\varrho$) von dem jeweiligen Einstrahlungsort befindet, austretendem Sekundärlicht (12) gemessen wird, und

   einen Auswerteschritt, bei dem eine Information über die Konzentration des Analyten in der biologischen Matrix mittels eines Auswertealgorithmus aus den bei den Detektionsmessungen gemessenen Intensitätsmeßwerten abgeleitet wird,

   bei welchem

   das Primärlicht mittels eines die Oberfläche (4) der biologischen Matrix (5) an dem Einstrahlungsort (10) kontaktierenden Lichtleitelementes (29) eingestrahlt wird,

   das Primärlicht an dem Einstrahlungsort (10) unter einem Einfallswinkel ($\alpha$) zu einem Lot (31) auf die Oberfläche (4) zwischen 5° und 85° schräg in die biologische Matrix (5) eingestrahlt wird und

bei mindestens zwei Detektionsmessungen der Meßabstand (e) zwischen dem jeweiligen Einstrahlungsort (10) und dem jeweiligen Detektionsort (33-40) höchstens der fünffachen mittleren freien Weglänge des in der biologischen Matrix propagierenden Lichtes entspricht.

3. Verfahren nach Anspruch 1 oder 2, bei welchem bei mindestens drei Detektionsmessungen der Meßabstand ($\varrho$) zwischen dem jeweiligen Einstrahlungsort (10) und dem jeweiligen Detektionsort (33-40) höchstens der fünffachen mittleren freien Weglänge des in der biologischen Matrix propagierenden Lichts entspricht.

4. Verfahren nach Anspruch 1 oder 2, bei welchem bei mindestens vier Detektionsmessungen der Meßabstand ($\varrho$) zwischen dem jeweiligen Einstrahlungsort (10) und dem jeweiligen Detektionsort (33-40) höchstens der fünffachen mittleren freien Weglänge des in der biologischen Matrix propagierenden Lichts entspricht.

5. Verfahren nach einem der vorhergehenden Ansprüche, bei welchem in dem Auswerteschritt ausschließlich Intensitätsmeßwerte verwendet werden, die aus Detektionsmessungen resultieren, bei denen der Meßabstand ($\varrho$) zwischen dem jeweiligen Einstrahlungsort (10) und dem jeweiligen Detektionsort (33-40) höchstens der zehnfachen mittleren freien Weglänge des in der biologischen Matrix (5) propagierenden Lichts entspricht.

6. Verfahren nach einem der vorhergehenden Ansprüche, bei weichem das Sekundärlicht (12) mittels eines die biologische Matrix an dem Detektionsort kontaktierenden Lichtleitelementes (30) detektiert wird.

7. Verfahren nach einem der vorhergehenden Ansprüche, bei welchem das Sekundärlicht (12) an dem Detektionsort (33-40) mit einer Detektionsrichtung (43) detektiert wird, die schräg zu der Oberfläche verläuft mit einem Winkel ($\beta$) zwischen 5° und 85°, bezogen auf ein Lot auf die Oberfläche.

8. Verfahren nach Anspruch 7, bei welchem die Detektionsrichtung (43) auf den Einstrahlungsort (10) zu orientiert ist.

9. Verfahren nach einem der vorhergehenden Ansprüche, bei welchem das Sekundärlicht an mehreren, jeweils eine Mehrzahl von Detektionsorten (49) umfassenden Detektionsfeldern (50,51,52) auf der Oberfläche der biologischen Matrix (5) detektiert wird, wobei die Detektionsrichtung bei der Mehrzahl der Detektionsorte (49) eines Detektionsfeldes (50,51,52) einheitlich, jedoch bei der Mehrzahl der Detektionsfelder unterschiedlich ist.

10. Verfahren nach Anspruch 1,
bei welchem der Auswertealgorithmus folgende Teilschritte einschließt:

a) Ermittlung der theoretischen Abhängigkeit der Intensität (I) des Sekundärlichts von dem Meßabstand ($\varrho$) und der optischen Absorption ($\mu_a$) mittels einer numerischen Simulation für einen typischen Wert ($\mu_{s,MC}$) der optischen Streuung,
b) Speicherung des Ergebnisses der Simulation in Form einer Lookup-Tabelle, durch die vorgegebenen Werten des Meßabstands ($\varrho$) und der optischen Absorption ($\mu_a$) jeweils ein Intensitätswert ($I(\varrho,\mu_a)$) zugeordnet wird,
c) Bestimmung des für die Lichtstreuung in der optischen Matrix charakteristischen Parameters ($\mu_s$) mittels eines Fit-Verfahrens, bei dem für eine Vielzahl unterschiedlicher Werte der den Lichttransport in der biologischen Matrix charakterisierenden Lichttransportparameter ($\mu_a,\mu_s$) die Abhängigkeit der Intensität (I) von dem Meßabstand ($\varrho$) berechnet und mit den bei den Detektionsmessungen gemessenen Intensitätsmeßwerten verglichen wird, um die Werte der Lichttransportparameter ($\mu_a,\mu_s$) zu bestimmen, bei denen eine optimale Übereinstimmung der rechnerisch bestimmten Abhängigkeit der Intensität (I) von dem Meßabstand ($\varrho$) mit den entsprechenden Intensitätsmeßwerten erreicht wird, wobei der Schritt c) folgende Teilschritte umfaßt:

c1) Berechnen eines Skalierungsfaktors (s) aus der Relation einer beliebigen optischen Streuung ($\mu_s$), zu der in dem Schritt a) verwendeten typischen optischen Streuung ($\mu_{s,MC}$),
c2) Umformen der unabhängigen Variablen ($\varrho,\mu_a$) der in dem Schritt b) gespeicherten Lookup-Tabelle mittels des Skalierungsfaktors (s),
c3) Berechnen der Abhängigkeit der Intensität (I) von der optischen Absorption ($\mu_a$) und dem Meßabstand ($\varrho$) bei dem gewählten Wert der optischen Streuung ($\mu_s$) mittels der umgeformten Variablen auf Basis der in der Lookup-Tabelle abgespeicherten Werte.

11. Verfahren nach Anspruch 10, bei welchem die Detektionsmessungen mittels eines tragbaren Analysegeräts durchgeführt werden, das ein Computersystem zur Durchführung eines Teils des Auswertealgorithmus einschließt, wobei die Schritte a) und b) außerhalb des tragbaren Analysegeräts mittels eines anderen Computersystems durchgeführt werden.

**12.** Meßkopf für eine Vorrichtung zur Durchführung des Verfahrens nach einem der vorhergehenden Ansprüche, enthaltend Lichteinstrahlungsmittel (7) zum Einstrahlen des Primärlichts an jeweils einem Einstrahlungsort und Detektionsmittel (16) zur Detektion des Sekundärlichts an jeweils einem Detektionsort, bei welchem
die Lichteinstrahlungsmittel (7) ein die Oberfläche der biologischen Matrix (5) an dem jeweiligen Einstrahlungsort (10) kontaktierendes Lichtleitelement (29) einschließen und so ausgebildet sind, daß das Primärlicht an dem Einstrahlungsort (10) unter einem Einfallswinkel ($\alpha$) zu einem Lot (31) auf die Oberfläche (4) zwischen 5° und 85° schräg in die biologische Matrix (5) einstrahlbar ist und ein Einstrahlungsort und zwei Detektionsorte oder zwei Einstrahlungsorte und ein Detektionsort so angeordnet sind, daß ihr Meßabstand (**Q**) höchstens 3,5 mm beträgt.

**13.** Vorrichtung zur Durchführung des Verfahren nach einem der Ansprüche 10 oder 11, insbesondere mit einem Meßkopf nach Anspruch 12, welche eine Signalverarbeitungs- und Auswerteeinheit einschließt, die programmtechnisch zur Durchführung des Schrittes c) ausgebildet ist.

**Claims**

**1.** Method for the selective determination of a light transport parameter which is characteristic of the light scattering in a biological matrix, comprising
at least three detection measurements, in each of which light is irradiated as primary light (9) into the biological matrix (5) at a light irradiation site (10) and then propagates in the biological matrix (5) along a light path (20-22), and an intensity measurement value of secondary light (12) is measured, the secondary light exiting at a detection site (33-40) that is located in the at least three detection measurements at different measuring distances (p) from the respective light irradiation site, and
an evaluation step, in which the light transport parameter which is characteristic of the light scattering in the biological matrix (5) is derived by means of an evaluation algorithm from the intensity measurement values measured in the detection measurements,
wherein
the primary light is irradiated by means of a light-guiding element (29) which contacts the surface (4) of the biological matrix (5) at the light irradiation site (10),
the primary light is irradiated into the biological matrix (5) at the light irradiation site (10) obliquely at a light irradiation angle between 5° and 85° relative to a normal (31) on the surface (4), and,
in at least two detection measurements, the measuring distance (p) between the respective light irradiation site (10) and the respective detection site (33-40) corresponds to no more than five times the mean free path length of the light propagating in the biological matrix.

**2.** Method for the determination of an analyte influencing the scattering of light in a biological matrix, in particular glucose, comprising
at least three detection measurements, in each of which light is irradiated as primary light (9) into the biological matrix (5) at a light irradiation site (10) and then propagates in the biological matrix (5) along a light path (20-22), and an intensity measurement value of secondary light (12) is measured, the secondary light exiting at a detection site (33-40) that is located in the at least three detection measurements at different measuring distances (p) from the respective light irradiation site, and
an evaluation step, in which an information concerning the concentration of the analyte in the biological matrix is derived by means of an evaluation algorithm from the intensity measurement values measured in the detection measurements,
wherein
the primary light is irradiated by means of a light-guiding element (29) which contacts the surface (4) of the biological matrix (5) at the light irradiation site (10),
the primary light is irradiated into the biological matrix (5) at the light irradiation site (10) obliquely at a light irradiation angle between 5° and 85° relative to a normal (31) on the surface (4), and,
in at least two detection measurements, the measuring distance (p) between the respective light irradiation site (10) and the respective detection site (33-40) corresponds to no more than five times the mean free path length of the light propagating in the biological matrix.

3. Method according to claim 1 or 2, wherein in at least three detection measurements, the measuring distance (p) between the respective light irradiation site (10) and the respective detection site (33-40) corresponds to no more than five times the mean free path length of the light propagating in the biological matrix.

4. Method according to claim 1 or 2, wherein in at least four detection measurements, the measuring distance (p) between the respective light irradiation site (10) and the respective detection site (33-40) corresponds to no more than five times the mean free path length of the light propagating in the biological matrix.

5. Method according to any one of the preceding claims, wherein only intensity measurement values resulting from detection measurements, in which the measuring distance (p) between the respective light irradiation site (10) and the respective detection site (33-40) corresponds to no more than ten times the mean free path length of the light propagating in the biological matrix (5), are included in the evaluation step.

6. Method according to any one of the preceding claims, wherein the secondary light (12) is detected by means of a light-guiding element (30) which contacts the biological matrix at the detection site.

7. Method according to any one of the preceding claims, wherein the secondary light (12) is detected at the detection site (33-40) at a detection direction (43) that runs oblique to the surface, at an angle ($\beta$) between 5° and 85° relative to a normal on the surface.

8. Method according to claim 7, wherein the detection direction (43) is oriented towards the light irradiation site (10).

9. Method according to any one of the preceding claims, wherein the secondary light is detected on the surface of the biological matrix (5) at a plurality of detection fields (50, 51, 52), each of which comprises a plurality of detection sites (49), the detection direction being the same for the plurality of detection sites of a detection field (50, 51, 52), but different for the plurality of detection fields.

10. Method according to claim 1,
    wherein the evaluation algorithm includes the following steps:

    a) determining by means of a numerical simulation for a typical value of optical scattering ($\mu_{s,MC}$) the theoretical dependence of the intensity (I) of the secondary light from the measuring distance (p) and the optical absorption ($\mu_a$);
    b) storing the result of the simulation as a lookup table by which an intensity value ($I(\rho,\mu_a)$) is assigned to predetermined values of the measuring distance (p) and optical absorption ($\mu_a$);
    c) determining the parameter ($\mu_s$) which is characteristic of the light scattering in the biological matrix by means of a fit procedure, in which for a plurality of different values of the light transport parameters ($\mu_a, \mu_s$) characterizing the light transport in the biological matrix the dependence of the intensity (I) on the measuring distance (p) is calculated and the result is compared to the intensity measurement values measured in the detection measurements in order to determine those values of the light transport parameters ($\mu_a, \mu_s$) at which an optimal agreement of the calculated dependence of the intensity (I) on the measuring distance (p) and the respective intensity measurement values is achieved, wherein step c) comprises the following substeps:

    c1) calculating a scaling factor (s) from the relation of any selected optical scattering value ($\mu_s$) to the typical optical scattering ($\mu_{s,MC}$) used in step a);
    c2) converting the independent variables (p, $\mu_a$) of the lookup table stored in step b) by means of the scaling factor (s);
    c3) calculating the dependence of the intensity (I) on the optical absorption ($\mu_a$) and the measuring distance (p) at the selected optical scattering value ($\mu_s$) by means of the converted variables on the basis of the values stored in the lookup table.

11. Method according to claim 10, wherein the detection measurements are performed by means of a portable analysis instrument which includes a computer system for performing a part of the evaluation algorithm, wherein steps a) and b) are performed outside of the analysis instrument by means of a different computer system.

12. Measuring head for a device for performing the method according to any one of the preceding claims, containing light irradiation means (7) for irradiating the primary light at one light irradiation site each and detection means (16) for detecting the secondary light at one detection site each, wherein

the light irradiation means (7) include a light-guiding element (29) that contacts the surface of the biological matrix (5) at the selected light irradiation site (10) and are arranged in such a manner that the primary light can be irradiated into the biological matrix obliquely at the light irradiation site (10) at a light irradiation angle (α) between 5° and 85° relative to a normal (31) on the surface (4), and

one light irradiation site and two detection sites or two light irradiation sites and one detection site are arranged such that their measuring distance (p) is at most 3,5 mm.

13. Device for performing the method according to any of claims 10 or 11, in particular by means of a measuring head according to claim 12, comprising a signal processing and evaluation unit programmed for performing step c).

**Revendications**

1. Procédé pour la détermination sélective d'un paramètre de transport de lumière caractéristique pour la diffusion de la lumière dans une matrice biologique, comprenant
   au moins trois mesures de détection, dans lesquelles de la lumière est irradiée en tant que lumière primaire (9) dans la matrice biologique (5) en un point d'irradiation (10), la lumière se propage dans la matrice biologique (5) le long d'un trajet optique (20-22), et l'on mesure une valeur d'intensité de la lumière secondaire (12) sortant en un point de détection (33-40) qui est situé dans les mesures de détection à des distances de mesure différentes (p) du point d'irradiation respectif, et
   une étape d'évaluation, dans laquelle le paramètre de transport de lumière caractéristique pour la diffusion de la lumière dans la matrice biologique (5) est dérivé, au moyen d'un algorithme d'évaluation, des valeurs d'intensité mesurées lors des mesures de détection,
   dans lequel
   la lumière primaire est irradiée à l'aide d'un élément conducteur de lumière (29) contactant la surface (4) de la matrice biologique (5) au point d'irradiation (10),
   la lumière primaire est irradiée obliquement dans la matrice biologique (5) au point d'irradiation (10) à un angle d'incidence (α) compris entre 5° et 85° par rapport à la perpendiculaire (31) sur la surface (4) et
   pour au moins deux mesures de détection, la distance de mesure (p) entre le point d'irradiation respectif (10) et le point de détection respectif (33-40) correspond tout au plus à cinq fois le parcours libre moyen de la lumière se propageant dans la matrice biologique.

2. Procédé pour la détermination d'un analyte qui influence la diffusion de la lumière dans une matrice biologique, notamment de glucose, comprenant
   au moins trois mesures de détection, dans lesquelles de la lumière est irradiée en tant que lumière primaire (9) dans la matrice biologique (5) en un point d'irradiation (10), la lumière se propage dans la matrice biologique (5) le long d'un trajet optique (20-22), et l'on mesure une valeur d'intensité de la lumière secondaire (12) sortant en un point de détection (33-40) qui est situé dans les mesures de détection à des distances de mesure différentes (p) du point d'irradiation respectif, et
   une étape d'évaluation, dans laquelle une information sur la concentration de l'analyte dans la matrice biologique est dérivée, au moyen d'un algorithme d'évaluation, à partir des valeurs d'intensité mesurées lors des mesures de détection,
   la lumière primaire est irradiée à l'aide d'un élément conducteur de lumière (29) contactant la surface (4) de la matrice biologique (5) au point d'irradiation (10),
   la lumière primaire est irradiée obliquement dans la matrice biologique (5) au point d'irradiation (10) à un angle d'incidence (α) compris entre 5° et 85° par rapport à la perpendiculaire (31) sur la surface (4) et
   pour au moins deux mesures de détection, la distance de mesure (p) entre le point d'irradiation respectif (10) et le point de détection respectif (33-40) correspond tout au plus à cinq fois le parcours libre moyen de la lumière se propageant dans la matrice biologique.

3. Procédé selon l'une des revendications 1 ou 2, dans lequel, pour au moins trois mesures de détection, la distance de mesure (p) entre le point d'irradiation respectif (10) et le point de détection respectif (33-40) correspond tout au plus à cinq fois le parcours libre moyen de la lumière se propageant dans la matrice biologique.

4. Procédé selon l'une des revendications 1 ou 2, dans lequel, pour au moins quatre mesures de détection, la distance de mesure (p) entre le point d'irradiation respectif (10) et le point de détection respectif (33-40) correspond tout au plus à cinq fois le parcours libre moyen de la lumière se propageant dans la matrice biologique.

**5.** Procédé selon l'une quelconque des revendications précédentes, dans lequel on utilise pour l'étape d'évaluation uniquement des valeurs d'intensité résultant de mesures de détection dans lesquelles la distance de mesure (p) entre le point d'irradiation respectif (10) et le point de détection respectif (33-40) correspond tout au plus à dix fois le parcours libre moyen de la lumière se propageant dans la matrice biologique (5).

**6.** Procédé selon l'une quelconque des revendications précédentes, dans lequel la lumière secondaire (12) est détectée au moyen d'un élément conducteur de lumière (30) contactant la matrice biologique au point de détection.

**7.** Procédé selon l'une quelconque des revendications précédentes, dans lequel la lumière secondaire (12) est détectée dans le point de détection (33-40) suivant une direction de détection (43) inclinée à l'oblique par rapport à la surface à un angle ($\beta$) compris entre 5° et 85° rapporté à la perpendiculaire de la surface.

**8.** Procédé selon la revendication 7, dans lequel la direction de détection (43) est orientée vers le point d'irradiation (10).

**9.** Procédé selon l'une quelconque des revendications précédentes, dans lequel la lumière secondaire est détectée sur la surface de la matrice biologique (5) dans plusieurs champs de détection (50,51,52) dont chacun comprend une pluralité de points de détection (49), la direction de détection étant la même pour la pluralité des points de détection (49) d'un champs de détection (50,51,52), mais différente pour la pluralité des champs de détection.

**10.** Procédé selon la revendication 1,
dans lequel l'algorithme d'évaluation inclut les étapes partielles suivantes:

a) détermination de la dépendance théorique entre l'intensité (I) de la lumière secondaire de la distance de mesure (p) et l'absorption optique ($\mu_a$), à l'aide d'une simulation numérique pour une valeur typique ($\mu_{s,MC}$) de la diffusion optique,
b) mémorisation du résultat de la simulation sous forme d'un tableau lookup qui assigne une valeur d'intensité (I($\rho,\mu_a$) à chacune des valeurs prédéterminées de la distance de mesure (p) et de l'absorption optique ($\mu_a$),
c) détermination du paramètre ($\mu_s$) caractéristique pour la diffusion de la lumière dans la matrice optique à l'aide d'un procédé FIT,

dans lequel la dépendance entre l'intensité (I) et la distance de mesure (p) est calculée pour un grand nombre de valeurs différentes des paramètres de transport de lumière ($\mu_a$, $\mu_s$) caractérisant le transport de lumière dans la matrice biologique et comparée avec les valeurs d'intensité mesurées lors des mesures de détection, afin de déterminer les valeurs des paramètres de transport de lumière ($\mu_a$, $\mu_s$), auxquelles la dépendance déterminée par calcul entre l'intensité (I) et la distance de mesure (p) correspond de façon optimale aux valeurs d'intensité mesurées respectives, l'étape c) comprenant les étapes partielles suivantes:

c1) calcul d'un facteur d'échelle (s) à partir de la relation entre une diffusion optique quelconque ($\mu s$) et la diffusion optique typique ($\mu_{s,MC}$) utilisée à l'étape a),
c2) conversion des variables indépendantes ($\rho,\mu_a$) du tableau lookup mémorisé à l'étape b) à l'aide du facteur d'échelle (s),
c3) calcul de la dépendance entre l'intensité (I), l'absorption optique ($\mu a$) et la distance de mesure (p) pour la valeur retenue de la diffusion optique ($\mu s$) à l'aide des variables converties sur la base des valeurs mémorisées dans le tableau lookup.

**11.** Procédé selon la revendication 10, dans lequel les mesures de détection s'effectuent à l'aide d'un appareil portable d'analyse doté d'un système informatique qui effectue une partie de l'algorithme d'évaluation, les étapes a) et b) étant effectuées en dehors de l'appareil portable d'analyse au moyen d'un autre système informatique.

**12.** Tête de mesure pour un dispositif destiné à réaliser le procédé selon l'une des revendications précédentes, comprenant des moyens d'irradiation de lumière (7) pour l'irradiation de la lumière primaire en un point d'irradiation et des moyens de détection (16) pour la détection de la lumière secondaire en un point de détection, dans lequel les moyens d'irradiation de lumière (7) incluent un élément conducteur de lumière (29) contactant la surface de la matrice biologique (5) au point d'irradiation (10) respectif, et sont configurés de sorte que la lumière primaire peut être irradiée au point d'irradiation (10) à un angle d'incidence ($\alpha$) par rapport à la perpendiculaire (31) de la surface (4) compris entre 5° et 85°, obliquement à la matrice biologique, et
le point d'irradiation et deux points de détection ou deux points d'irradiation et un point de détection sont disposés de sorte que leur distance de mesure (p) est de 3,5 mm au maximum.

13. Dispositif pour la mise en oeuvre du procédé selon l'une des revendications 10 ou 11, en particulier avec une tête de mesure selon la revendication 12, comprenant une unité de traitement des signaux et d'évaluation qui est dotée d'un logiciel apte à apte à effectuer l'étape c).

Fig. 1

Fig. 2

Fig.3

Fig.4

Fig.5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0659055 B1
- EP 0760091 B1
- EP 0774658 A2
- DE 10110599
- US 5630423 A
- US 5893364 A

### In der Beschreibung aufgeführte Nicht-Patentliteratur

- **T.J. FARRELL et al.** A diffusion theory model of spatially resolved, steady-state diffuse reflectance for the noninvasive determination of tissue optical properties in vivo. *Med. Phys.,* 1992, vol. 19, 879-888
- **R.C. HASKELL et al.** Boundary conditions for the diffusion equation in radiative transfer. *J. Opt.Soc.Am A,* 1994, vol. 11, 2727-2741
- **G. MARQUEZ et al.** White light oblique incidence reflectometer for measuring absorption and reduced scattering spectra of tissue-like turbid media. *OPTICS EXPRESS,* 1997, 454-460
- **S.A. PRAHL et al.** A Monte Carlo Model of light propagation in tissue. *SPIE Institute Series,* 1989, vol. IS 5, 102-111